(19) Europäisches Patentamt
European Patent Office
Office européen des brevets

(11) **EP 1 629 816 A1**

(12) **EUROPEAN PATENT APPLICATION**

(43) Date of publication:
**01.03.2006 Bulletin 2006/09**

(51) Int Cl.:
*A61H 1/00* (2006.01)   *A61H 37/00* (2006.01)
*A61B 5/053* (2006.01)

(21) Application number: **05018706.1**

(22) Date of filing: **29.08.2005**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **30.08.2004 JP 2004250656**

(71) Applicant: **Omron Healthcare Co., Ltd. Kyoto 615-0084 (JP)**

(72) Inventor: **Kasai, Eiji c/o KETEC CO., Ltd. Kyoto 617-0002 (JP)**

(74) Representative: **Kilian, Helmut Wilhelms, Kilian & Partner Patentanwälte Eduard-Schmid-Strasse 2 81541 München (DE)**

(54) **Massager**

(57)     A massager includes a back rest portion, massage heads (402a-402d), a treatment section (40), a sensor section (100), and a control section (13). The massage heads are provided to the back rest portion, and massage a user. The treatment section moves a pair of massage head arms (403R,403L) supporting the massage heads, in association with a massage action. A sensor section (113) senses a bio-signal of the user from an impedance variance between the pair of massage head arms. The control section controls the massage action of massage heads, and makes a comparison between a signal corresponding to the bio-signal and a predetermined reference value, thereby to determine the presence or absence of the user.

## FIG. 1

EP 1 629 816 A1

**Description**

Background of the invention

Field of the invention

The present invention relates to a massager. More specifically, the invention relates to a massager that uses a sensor for sensing, for example, a shoulder position and a sitting posture of a user.

Description of the related art

In many conventional massagers, a massage action is performed in accordance with a shoulder position of a user. In addition, there are massagers of the type that performs start/stop action in accordance with the presence or absence of the sitting posture of a user.

Means of the type determining the shoulder position in the massager and the presence or absence of the sitting posture of the user include those described herebelow.

Japanese Patent No. 3128260 discloses a massager that sensing a massage site of the shoulders of a user in such a manner that a strain gauge is used to detect the pressure of a roller onto the massage site or to detect the angle of an arm provided with the roller.

Japanese Unexamined Patent Application Publication No. 2003-102799 discloses a massager using a plurality of infrared-light receiving photosensors deployed at heights different from one another to a front face of a back rest. In this case, the shoulder position of a user is detected from the position of the photosensors blocked off by the user from an infrared ray emitted from a remote control unit.

Japanese Unexamined Patent Application Publication No. 11-42263 discloses a massager that detects the presence or absence of a person and detects the shoulder position of the person in accordance with electrostatic capacitance detected by a plurality of detectors provided to a treatment frame assembly.

Japanese Unexamined Patent Application Publication No. 2001-269380 discloses a massager that controls the massage action in accordance with a signal detected by pressure sensors mounted on an outer circumferential surface of a treatment applicator.

However, according to the massager disclosed in Japanese PatentNo. 3128260, straingauges, angle sensors are necessary, the structure of massage unit becomes complicate, thereby increase costs for sensing.

Further, according to the massager disclosed in Japanese Unexamined Patent Application Publication No. 2003-102799, the resolution is determined in accordance with the number of photosensors, so that the cost is increased to enhance the resolution. In addition, depending on the operation position of the remote control unit, error occurs in the designation of the shoulder position.

Further, according to the massager disclosed in Japanese Unexamined Patent Application Publication No. 11-42263, since the resolution is determined in accordance with the number of detectors, the cost is increased to enhance the resolution.

Further, according to the massager disclosed in Japanese Unexamined Patent Application Publication No. 2001-269380, since a plurality of pressure sensors are necessary, the structure becomes complicate, and the cost is increased. Further, nonuniformity in sensing dependent on the sitting manner of the user is increased.

SUMMARY OF THE INVENTION

The present invention is made in view of the related art described above. Accordingly, an object of the present invention is to provide a massager that has a simple structure, but accurately senses the shoulder position and the presence or absence of the sitting posture of the user.

To achieve the above-mentioned object, the present invention provides a massager comprising:

a back rest portion that supports a back side of a user;
treatment applicators that are provided to the back rest portion and that massage the user;
a treatment section that moves a pair of electrodes supporting the treatment applicators, in association with a massage action;
a bio-signal sensor that senses a bio-signal of the user from an impedance variance between the pair of electrodes; and
a control section that controls the massage action of the treatment applicators in accordance with the bio-signal, wherein
the bio-signal sensor includes:

an oscillation section that supplies a high frequency signal;
the pair of electrodes that are disposed to allow the user to near or contact and that are provided to receive the high frequency signal from the oscillator section;
a sensor section that senses the impedance variance occurring between the electrodes having received the high frequency signal when the user has neared and contacted the electrodes; and
a signal processor section that processes a signal corresponding to the sensed impedance variance into a desired signal corresponding to the bio-signal,
wherein the control section makes a comparison between the signal corresponding to the bio-signal and a predetermined reference value, thereby to determine presence or absence of a sitting posture of the user.

According to the configuration, with the pair of electrodes supporting the treatment applicators, the presence or absence of the sitting posture of the user can be more simply sensed. In addition, additional sensing-dedicated components are not necessary, nor are specific processes necessary on the back rest portion, therefore making it possible to provide low cost massagers having a simple configuration.

Preferably, there is provided a massager, wherein when the signal corresponding to the bio-signal is not greater than or equal to the predetermined reference value during movement of the treatment applicators in a set range, the control section controls the massage action with the treatment applicators to not start.

According to the configuration described above, even in the event massage action is started by the user by accident, when the sitting posture of the user is not present, subsequent action of the massager can be automatically stopped, thereby to enable unnecessary massage action to be prevented.

Preferably, there is provided a massager, wherein when the signal corresponding to the bio-signal is not greater than or equal to the predetermined reference value during movement of the treatment applicators in a set range, the control section controls the massage action being performed with the treatment applicators to stop.

According to the configuration described above, with the use of the pair of electrodes supporting the treatment applicators, sensing can be performed while the massage action is being performed, the presence or absence of the sitting posture of the user can be sensed in an intermediate stage of automatic massage action. Consequently, even when the user has left the seat portion, subsequent massage action can be stopped automatically, thereby to enable unnecessary massage action to be prevented.

Preferably, there is provided a massager, wherein the electrodes are pivotable with respect to the treatment section, and are arms each supporting a plurality of the treatment applicators; and the plurality of treatment applicators are provided to be aligned substantially parallel to a movement direction of the treatment section in a manner to sandwich a pivoting center of the arm.

According to the configuration, among the plurality of treatment applicators supported to the respective arm, when one of the treatment applicators provided on the side of the moving direction of the treatment section abuts the user, reactive forces from the user act on the abutted one treatment applicator, thereby to pivotally move the arm. Thereby, the one treatment applicator and the other treatment applicator, which is provided opposite the one treatment applicator, move closer to the user. Consequently, the bio-signal from the user can be accurately sensed, thereby making it possible to determine the presence or absence of the sitting posture even more accurately.

Further, according to another aspect of the present invention, there is provided a massager comprising:

a back rest portion that supports a back side of a user;
treatment applicators that are provided to the back rest portion and that massage the user;
a treatment section that moves a pair of electrodes supporting the treatment applicators, in association with a massage action;
an elevation section that elevationally move the treatment section within the back rest portion;
a bio-signal sensor that senses a bio-signal of the user from an impedance variance between the pair of electrodes; and
a control section that controls the massage action of the treatment applicators in accordance with the bio-signal, wherein
the bio-signal sensor includes:

an oscillator section that supplies a high frequency signal;
the pair of electrodes that are disposed to allow the user to near or contact and that are provided to receive the high frequency signal from the oscillator section;
a sensor section that senses the impedance variance occurring between the electrodes having received the high frequency signal when the user has neared and contacted the electrodes; and
a signal processor section that processes a signal corresponding to the sensed impedance variance into a desired signal corresponding to the bio-signal,

wherein the control section causes the treatment section to move and detects an extreme value of the signal corresponding to the bio-signal in a predetermined range, thereby to determine a shoulder position of the user.

Preferably, there is provided a massager, wherein the extreme value is an initial peak value.

According to these configuration, the shoulder position is automatically determined without requiring the user to set the shoulder position. Consequently, the accurate massage action can be performed without excessive burden being imposed on the user.

Preferably, there is provided a massager, wherein when the initial peak value is not greater than or equal to a predetermined reference value, the control section does not determine the shoulder position of the user.

The configuration enables preventing a case where the shoulder position is incorrectly determined in an initial measurement zone wherein a signal corresponding to the bio-signal obtained from the biological signal sensor vertically oscillates, and hence the massager performs the massage action in accordance with the incorrect shoulder position.

Preferably, there is provided a massager, wherein when a tilt of the back rest portion is altered, the control section again determines the shoulder position and controls the massage action with the treatment applicators in accordance with the shoulder position again determined.

According to the configuration, even when the tilt of the back rest portion is altered (i . e . , reclining), the shoulder position is again sensed, thereby to enable an appropriate massage action to be performed.

Preferably, there is provided a massager, wherein when a tilt of the back rest portion is altered, the control section controls the massage action with the treatment applicators in accordance with the shoulder position corrected corresponding to the tilt.

According to the configuration, the treatment section need not be moved to an initial position to again sense the shoulder position, so that the time of a massage treatment can be reduced.

Preferably, there is provided a massager, wherein after a predetermined time has elapsed since the control section determined the shoulder position, the control section again determines the shoulder position and controls the massage action with the treatment applicators in accordance with the shoulder position again determined.

According to the configuration described above, the shoulder position can be accurately determined at all times, and an optimal massage action can be performed.

Preferably, there is provided a massager, wherein the electrodes are pivotable with respect to the treatment section, and are arms each supporting a plurality of the treatment applicators; and the plurality of treatment applicators are provided to be aligned substantially parallel to a movement direction of the treatment section in a manner to sandwich a pivoting center of the arm.

According to the configuration, among the plurality of treatment applicators supported to the respective arm, when one of the treatment applicators provided on the side of the moving direction of the treatment section abuts the user, reactive forces from the user act on the abutted one treatment applicator, thereby to pivotally move the arm. Thereby, the one treatment applicator and the other treatment applicator, which is provided opposite the one treatment applicator, move closer to the user. Consequently, the bio-signal from the user can be accurately sensed, thereby making it possible to determine the shoulder position of the user even more accurately.

Preferably, there is provided a massager, wherein the sensor section generates and supply a signal that represents a variance in a level of a reflected wave of the high frequency signal and that corresponds to the impedance variance occurring between the electrodes in correspondence to a body type of the user.

Preferably, there is provided a massager, wherein the sensor section outputs reflection wave signals corresponding to real and imaginary impedance variances occurring between the electrodes in correspondence to a body type of the user.

Preferably, there is provided a massager, wherein the frequency of the high frequency signal is in a range of from 10 MHz to 300 MHz.

According to the present invention, it is possible to realize a massager that has a simple structure, and accurately senses the shoulder position and the presence or absence of the sitting posture of the user.

Brief description of the drawings

FIG. 1 is a schematic block diagram showing a sensor section included in an electrical configuration of a massager according to an embodiment of the present invention;

FIG. 2 is an overall front elevation view showing schematic configuration of the massager according to the embodiment;
FIG. 3 is an overall side view showing schematic configuration of the massager according to the embodiment;
FIG. 4 is a front elevation view of elevating treatment unit according to the embodiment;
FIG. 5 is rearside elevation view of the elevating treatment unit according to the embodiment;

FIG. 6 is a perspective view of a treatment section of the massager according to the embodiment;
FIG. 7 is a schematic block diagram of an electrical configuration of a massager according to the embodiment;
FIG. 8 is a flow diagram showing operation of the massager, according to a first embodiment example;
FIG. 9 is a flow diagram showing operation of the massager, according to a second embodiment example;
FIG. 10 is a graph showing an example of variances in output obtained from the sensor section in the massager according to the embodiment;
FIG. 11 is a flow diagram showing operation of the massager, according to a third embodiment example;
FIG. 12 is a flow diagram showing operation of the massager, according to a fourth embodiment example;
FIG. 13 is a flow diagram showing operation of the massager, according to a fifth embodiment example; and
FIG. 14 is a flow diagram showing operation of the massager, according to a sixth embodiment example.

Detailed description of the preferred embodiments

With reference to the accompanying drawings, the preferred embodiments for carrying out the present invention will be described in detail hereinbelow. However, unless specifically described, it is not intended that the scope of the present invention is not limited to dimensions, materials, shapes, and functions of components, and relative deployment of the components described below in the embodiments. In addition, unless newly described, materials, shapes, functions, and the like of members initially described remains similar or identical for those subsequently described.

(Overall Configuration of Massager)

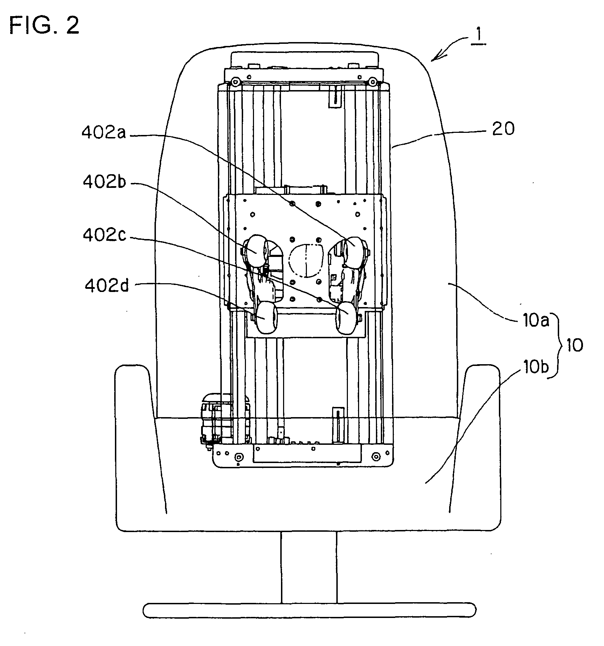

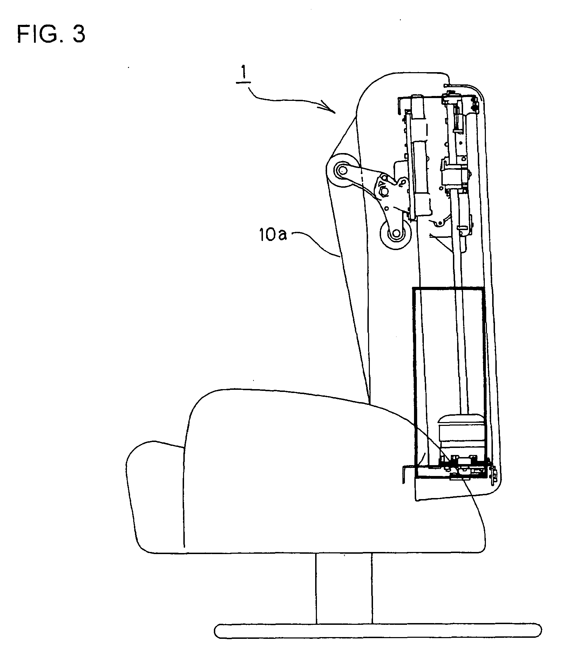

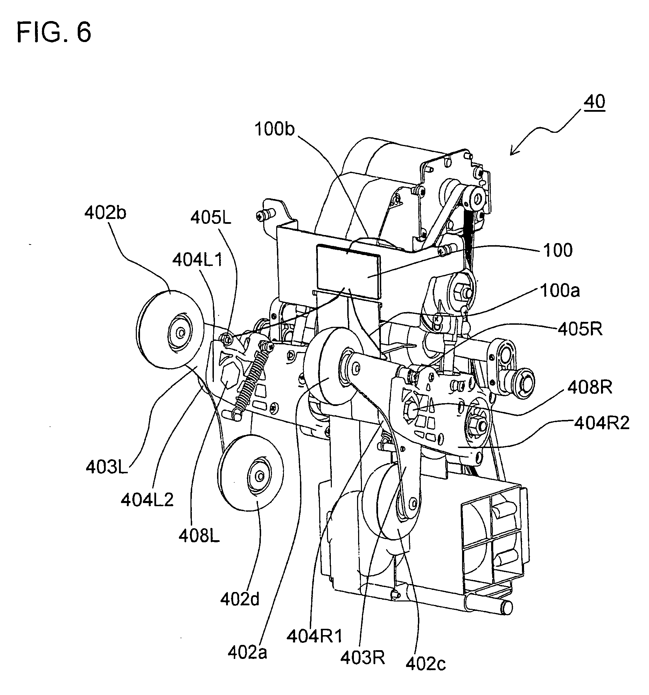

FIG. 2 is an overall front elevation view showing an schematic configuration of a massager 1 capable of preferably carrying out the present invention. FIG. 3 is an overall side view showing an schematic configuration of the massager 1, which is capable of preferably carrying out the present invention.
The massager 1 includes a reclinable chair 10 and an elevating treatment unit 20. The chair 10 consist of a back rest portion 10a for supporting the back face of a user, and a seat portion 10b, wherein elevating treatment unit 20 is assembled into the back rest portion 10a.
The elevating treatment unit 20 includes massage heads 402a to 402d (treatment applicators) provided to protrude toward the side of a surface covered by a cover sheet of the back rest portion 10a. Human body massaging is done by the massage head 402a to 402d.
The massage heads 402a to 402d are provided to the back rest portion 10a, thereby to massage the user. More specifically, the massage heads 402a to 402d are separated into the massage heads 402a and 402b (first treatment applicators) provided in a pair in upper right and left portions along the spine direction, and the massage heads 402c and 402d (second treatment applicators) provided in a pair in lower right and left portions below the massage heads 402a and 402b.

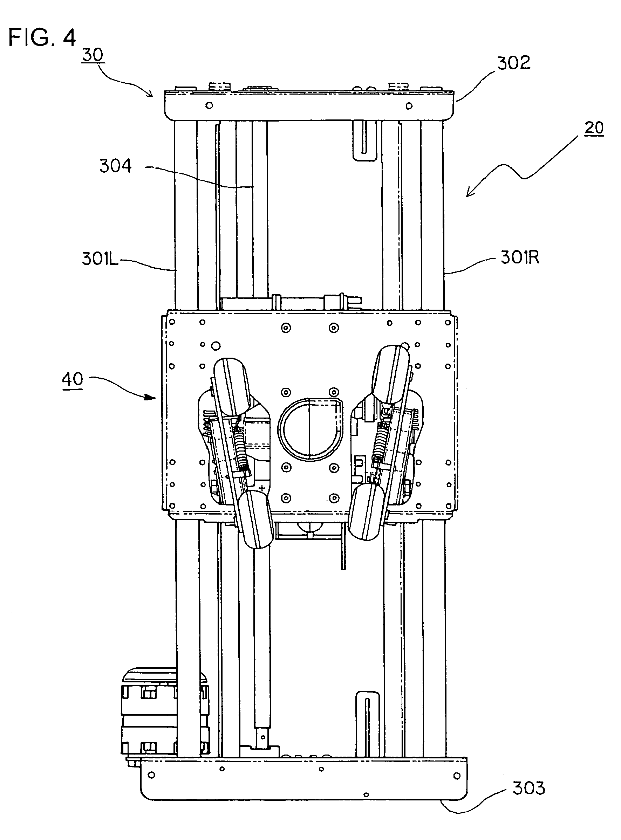

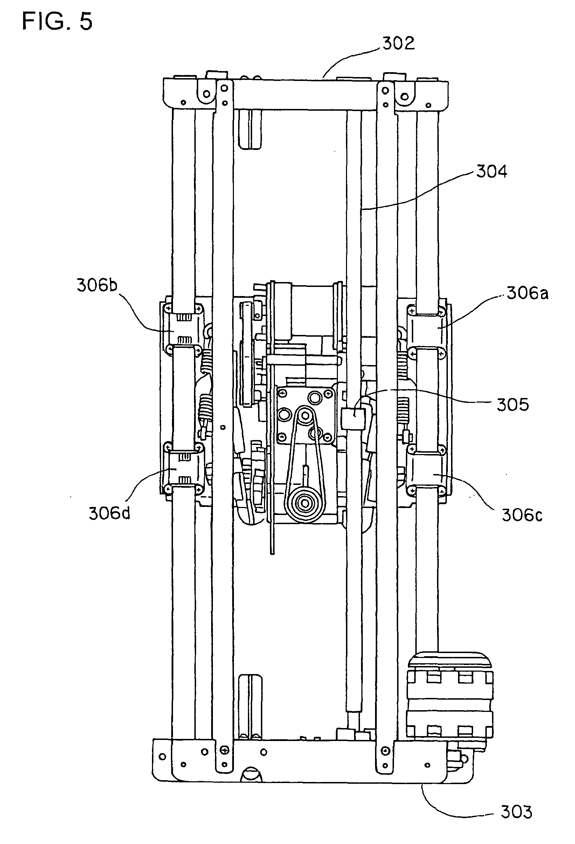

FIG. 4 is a front elevation view of the elevating treatment unit. FIG. 5 is a rearside elevation view of the elevating treatment unit.
The elevating treatment unit 20 includes an elevation section 30 and a treatment section 40 and thereby to move the arms 403R and 403L in association with the massage action. The arms 403R and 403L functions as a pair of electrodes that support the treatment applicators (refer to FIG. 6) . With reference to in FIG. 4, the elevation section 30 includes a pair of guide pipes 301R and 301L that are each circular in cross section and are disposed along the back rest portion 10a; elevation-dedicated threaded shaft 304 disposed parallel to and between the two guide pipes 301R and 301L; and a pair of upper and lower guide pipe holders 302 and 303 disposed perpendicular to the guide pipes 301R and 301L.
The guide pipes 301R and 301L are secured to guide pipe holders 302 and 303. The elevation-dedicated threaded shaft 304 is rotatably supported.
With reference to FIG. 5, the treatment section 40 is supported by elevation guides 306a, 306b, 306c, 306d supported axially movably to the guide pipes 301R and 301L and by an elevation nut holder (not shown) that holds an elevation nut 305 engaging an outer circumference of the elevation-dedicated threaded shaft 304.
In conjunction with the elevation nut 305 being vertically driven by the rotation of the elevation-dedicated threaded shaft 304, the elevation nut holder and the treatment section 40 supporting the elevation nut holder are vertically moved along the guide pipes 301R and 301L.

(Schematic Configuration of Treatment Section)

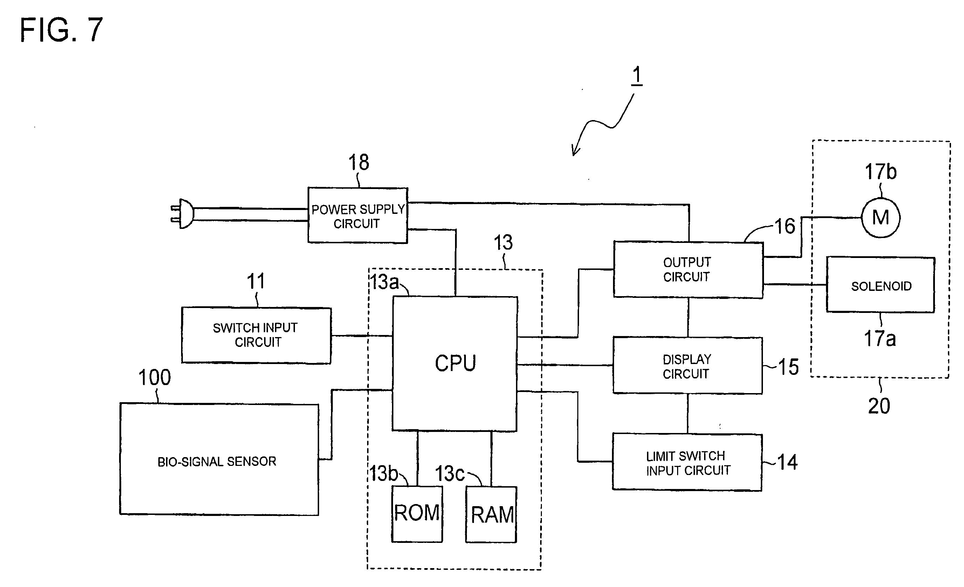

The schematic configuration of the treatment section 40 will be described hereinbelow. FIG. 6 is a perspective view of the treatment section 40 of the massager 1 capable of preferably carrying out the present invention.
The treatment section 40 has the four massage heads 402a to 402d, the massage head arms 403R and 403L which is substantially "V" shape, a bio-signal sensor 100 (hereinafter referred to as "sensor section"), and a control section 13 (refer to FIG. 7). The sensor section 100 oscillates a signal of a predetermined frequency, and senses a bio-signal emitted from the user from an impedance variance between the massage head arms 403R and 403L. The control section 13 controls the massage action of the massager 1 including the treatment section 40 in accordance with the bio-signal output from the sensor section 100.

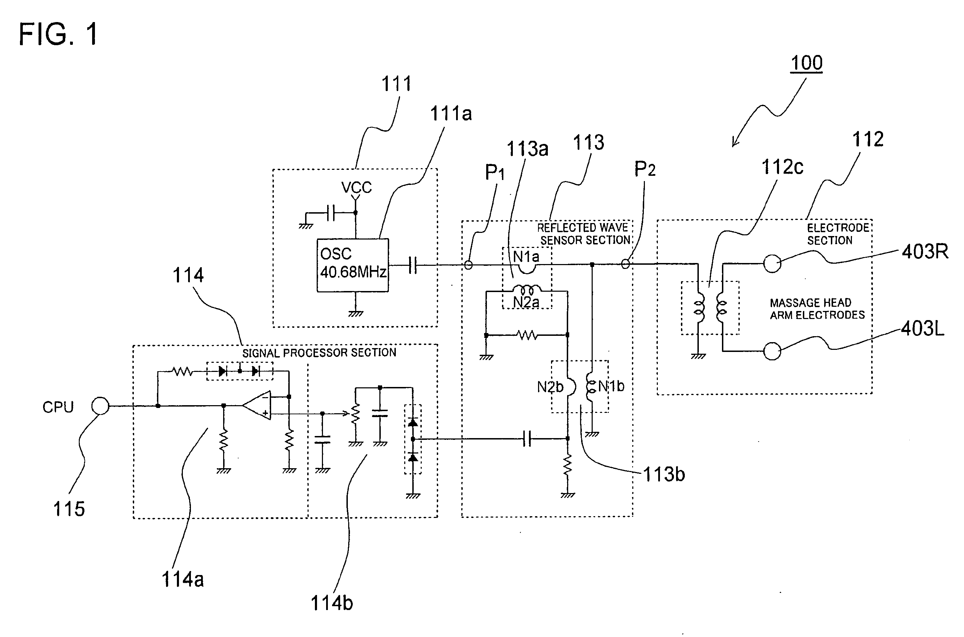

The massage head arms 403R and 403L respectively function as electrodes for sensing the shape and presence or absence of a part of the human body. The arms 403R and 403L are connected to a reflected wave sensor section 113 (refer to FIG. 1) of the sensor section 100 through a pair of power feed lines 100a provided to supply high frequency signals from an oscillator section 111 (refer to FIG. 1) . Ends of the power feed line 100a are connected by, being screwed or soldered, to the respective massage head arms 403R and 403L.

The massage head arms 403R and 403L have end portions respectively rotatably journaling two of the massage heads 402a to 402d.

A base end portion of the massage head arm 403R is sandwiched by members 404R1 and 404R2 of an arm support member 404R, and is supported by a pivotal shaft 408R. A base end portion of the massage head arm 403L is sandwiched by members 404L1 and 404L2 of the arm support member 404L, and is supported by a pivotal shaft 408L. Thereby, the massage head arms 403R and 403L are rotatable with respect to the treatment section 40.

The plurality of massage heads 402a to 402d provided to the respective massage head arms 403R and 403L are provided substantially parallel to the movement direction of the treatment section 40.

In addition, stoppers 405R and 405L for stopping the pivotal movements are provided to the massage head arms 403R and 403L, respectively.

The massage head arms 403R and 403L are each pivotally movable with respect to the treatment section 40, and each support plural ones of massage heads 402a to 402d. The plural ones of the massage heads 402a to 402d are preferably aligned and provided substantially parallel to the movement direction of the treatment section 40 with a pivoting center of each of the massage head arm 403R and 403L being interposed.

According to the configuration, among the plurality of massage heads 402a to 402d supported to the massage head arms 403R and 403L, when the one-side massage heads 402c and 402d provided on the side of the moving direction of the treatment section 40 abut a user, reactive forces from the user act on the abutted one-side massage heads 402c and 402d, thereby to pivotally move the massage head arms 403R and 403L. Thereby, the one-side massage heads 402c and 402d and the other-side massage heads 402a and 402b provided on the opposite side move even closer to the user, consequently making it possible to accurately sense the bio-signal from the user.

(Control circuit of Massager)

FIG. 7 is a schematic block diagram showing an electrical configuration of the massager 1 according to the present invention.

With reference to FIG. 7, the massager 1 includes, mainly, a switch input circuit 11, the sensor section (bio-signal sensor) 100, the control section 13 for controlling the operation of the massager 1, a limit switch input circuit 14, a display circuit 15, an output circuit 16, the elevating treatment unit 20, and a power supply circuit 18.

The control section 13 includes a central processing unit (CPU) 13a, a read-only memory (ROM) 13b, and a random access memory (RAM) 13c. The elevating treatment unit 20 is provided to the back rest portion 10a, and includes a solenoid 17a and a motor 17b.

Although not shown, in addition to a power switch, the switch input circuit 11 includes key switches for specifying various motions of elevating treatment unit 20, such as, for example, kneading (massaging), patting, ascension, and descension.

The sensor section 100 is a bio-signal sensor for sensing bio-signals of the user, and generates a bio-signal of, for example, the frequency of pulses or the frequency of respirations, thereby to supply the signal to the CPU 13a of the control section 13. Such a bio-signal is variable depending on the difference in the user's body type, muscles, fat, or the like, and is used such that the body type, body fat percentage, and the like of the user is indirectly measured and computed through the signal processing of the bio-signal.

In the event of using the massager 1, when the power switch (not shown) of a switch input circuit 11 is turned on by a user, CPU 13a responsively activates the power supply circuit 18, and the power supply circuit 18 in turn supplies power to respective sections of the massager 1.

In response to a specification given by the user through the switch input circuit 11, the CPU 13a generates a signal for driving the elevating treatment unit 20 and supplies the signal to the output circuit 16. In response to the signal, the output circuit 16 drives the solenoid 17a and the motor 17b, which constitute the elevating treatment unit 20, thereby to implement a motion specified by the user.

In addition, the CPU 13a drives the display circuit 15, and displays necessary information such as contents specified by the user to be viewable by the user.

The limit switch input circuit 14 defines a range of the vertical movement of the treatment section 40 in the elevating treatment unit 20 on the back rest portion 10a. In the event that the treatment section 40 ascends to reach the position of an upper limit or descends to reach the position of a lower limit, a limit switch is driven, thereby to stop the vertical movement of the treatment section 40.

The overall action of the massager 1 shown in FIG. 2 in accordance with the bio-signal sensed by the treatment section 40 according to the present invention will be described below.

(Circuit Configuration of Sensor Section)

FIG. 1 is a schematic block diagram showing a sensor section included in an electrical configuration of the massager 1 shown in FIG. 7.

As shown in FIG. 1, the sensor section 100 includes the oscillator section 111, an electrode section 112, the reflected wave sensor section 113, a signal processor section 114, and an output terminal 115. More specifically, the electrode section 112 includes the massage head arms 403R and 403L, which functions as electrodes, and a transformer 112c.

The pair of massage head arms 403R and 403L is disposed so as to enable a user to near or contact, is provided to receive high frequency signals from the oscillator section 111, and is connected to the treatment section 40.

Operation of the sensor section 100 will be outlined herebelow. The oscillator section 111 supplies a high frequency signal, and the massage head arms 403R and 403L receives the high frequency signal therefrom through the transformer 112c being used for isolation and real value conversion. In more specific, both ends of a primary coil of a transformer 112c are, respectively, coupled to the reflected wave sensor section 113 and the ground potential, and both ends of a secondary coil are, respectively, connected to the massage head arms 403R and 403L.

As described below, the impedances in the massage head arms 403R and 403L are fluctuated depending on the human body movement, shape, and the like. As such, also the energy of the high frequency signal being consumed at the electrodes is fluctuated depending on the human body movement, shape, and the like. Accordingly, a variance in energy of the high frequency signal remained unconsumed at the electrode is sensed as a variance in reflection level by the reflected wave sensor section 113. The sensed variance in the reflection wave level is amplified and signal-processed by the signal processor section 114, and is then output in the form of a bio-signal from the output terminal 115. The output bio-signal is supplied to the CPU 13a, which controls the massager 1 (refer to FIG. 7).

As shown in FIG. 2, the massage head arms 403R and 403L are disposed on the reverse side of the surface member of the back rest portion 10a of the massager 1, and the sensor section 100 integrally configured of other circuit elements is disposed in the treatment section 40 provided inside of the back rest portion 10a (refer to FIG. 6). The aforementioned other circuit elements are, as shown in FIG. 1, the transformer 112c, oscillator section 111, reflected wave sensor section 113, and signal processor section 114. Although not shown, the bio-signal output from the output terminal 115 of the sensor section 100 is supplied to the CPU 13a (refer to FIG. 7) of the control section 13 disposed inside of the massager 1.

A configuration of the oscillator section 111 of the sensor section 100 shown in FIG. 1 will nowbe described herebelow.

The oscillator section 111 has an oscillator 111a, such as a crystal oscillator, thereby constituting an oscillator circuit for outputting high frequency pulse signals. The oscillator section 111 further has two lowpass filters (not shown) for transforming pulse signals into sinusoidal waves, and an attenuator (not shown).

Such a configuration is made for the reason that, only in the event a sinusoidal wave signal is output as a high frequency output of the oscillator section 111, when an impedance match is recognized between the oscillator section 111 and the massage head arm 403R, 403L, the overall energy of a sinusoidal wave component is consumed at the electrode to cause the level of the reflected wave from the electrode to be zero. In other words, unless the output is a sinusoidal wave signal, energy of a non-sinusoidal wave component is reflected from an all-time loaded electrode, regardless of impedance matching.

For the high frequency component, the frequency thereof is appropriately selectable, but the frequency is preferably within a range of from 10 MHz to 300 MHz. In the present embodiment, the frequency is set to 40.68 MHz, but is not limited thereto. For example, 13.56 MHz and 27.12 MHz are preferable frequencies as well.

Aconfigurationof the reflected wave sensor section 113 of the sensor section 100 shown in FIG. 1 will be described herebelow.

The reflected wave sensor section 113 has terminals $P_1$ and $P_2$ and M coupled circuits 113a and 113b each having a well-known configuration. An oscillation output of the oscillator section 111 is coupled to the terminal $P_1$, an input end of the electrode section 112 (one end of the primary coil of the transformer 112c) is coupled to the terminal $P_2$.

The M coupled circuit 113a is configured of a primary coil N1a inserted between the terminals $P_1$ and $P_2$, a secondary coil N2a, and a resistor R parallel connected to both ends of the secondary coil N2a. The M coupled circuit 113b is configured of a primary coil N1b inserted between the terminal $P_2$ and a ground potential, and a secondary coil N2b. The secondary coils N2a and N2b are each series connected to the ground potential and an output end of the reflected wave sensor section 113.

Principles for sensing of the bio-signal by the reflected wave sensor section 113 will be described in detail herebelow.

By way of example, it is assumed that in the chair type massager 1 shown in FIG. 2, a user is taking a correct sitting posture along the back rest portion 10a. More specifically, it is assumed that the user is taking the correct sitting posture so that the shoulder position of the user is positioned near the massage head arm 403R, 403L.

When the high frequency sinusoidal wave signal supplied from the oscillator section 111 is supplied to the electrodes, a flow of a high frequency wave current occurs between the two massage head arms 403R and 403L through the surface member of the massager 1, the clothing of the user, and human body tissue (primarily, a fat component) of the user.

In more exact words, when the user nears or contacts the massage head arms 403R and 403L, the reflected wave sensor section 113 senses an impedance variance occurring between the massage head arms 403R and 403L functioning as the electrode receiving the high frequency signal.

The factor such as a distance variance between an electrode surface and a human body surface depending on the human body movement or a distance variance from the respective electrode surface to the human body tissue depending on the human body type corresponds to a variance in an imaginary component of a high frequency impedance in the electrode. In addition, the deformation in the human body tissue depending on the human body movement corresponds to a variance in a real component of the high frequency impedance.

The former distance variance and the latter human body tissue variance cyclically occur depending on the shape on the back side of the user who is taking a sitting posture. Consequently, a high frequency impedance variance corresponding to the human body movement or human body type of the user occurs at the respective electrode.

In other words, the reflected wave sensor section 113 generates and supplies a signal that represents a variance in the level of the reflected wave of the high frequency signal and that corresponds to the impedance variance occurring between the massage head arms 403R and 403L in correspondence to the body type of the user.

Alternatively, the reflected wave sensor section 113 may be configured to output reflection wave signals corresponding to real and imaginary impedance variances occurring between the massage head arms 403R and 403L in correspondence to the body type of the user.

In general, an impedance Z is expressed as follows:

$$Z = R + j(\omega L - (1/\omega C)) \qquad (equation\ 1)$$

where,

R: resistance;
L: inductance;
C: capacitance; and
$\omega$: angular frequency

The high frequency signal supplied from the oscillator section 111 is all consumed at the electrodes if the impedance match is attained between the massage head arms 403R and 403L. However, the energy consumed at the electrodes is varied corresponding to the impedance variance between the electrodes, and residual energy is returned as a reflected wave from the electrode section 112 to the oscillator section 111 through the reflected wave sensor section 113. The M coupled circuits 113a and 113b of the reflected wave sensor section 113 form directional couplers, whereby a part of the energy unconsumed at the electrodes is taken out and supplied as a reflected wave output to the signal processor section 114 at a rear stage.

The M coupled circuit using the coil is just a well-known example of directional coupler, and the reflected wave sensor section 113 can be alternately configured by using, for example, capacitors and microstrip lines.

A configuration of the signal processor section 114 of the sensor section 100 shown in FIG. 1 will be described hereinbelow.

The signal processor section 114 has an amplifier 114a and a filter 114b. As described above, a signal indicative of the variance in the level of the reflected wave at the massage head arms 403R and 403L is supplied to the signal processor section 114 from the reflected wave sensor section 113. The signal is then amplified in the signal processor section 114 and is passed through the filter wherein an appropriate constant is set, whereby a signal corresponding to the body type of the user can be output.

In other words, the signal processor section 114 processes the signal corresponding to the sensed impedance variance into a signal corresponding to a desired bio-signal.

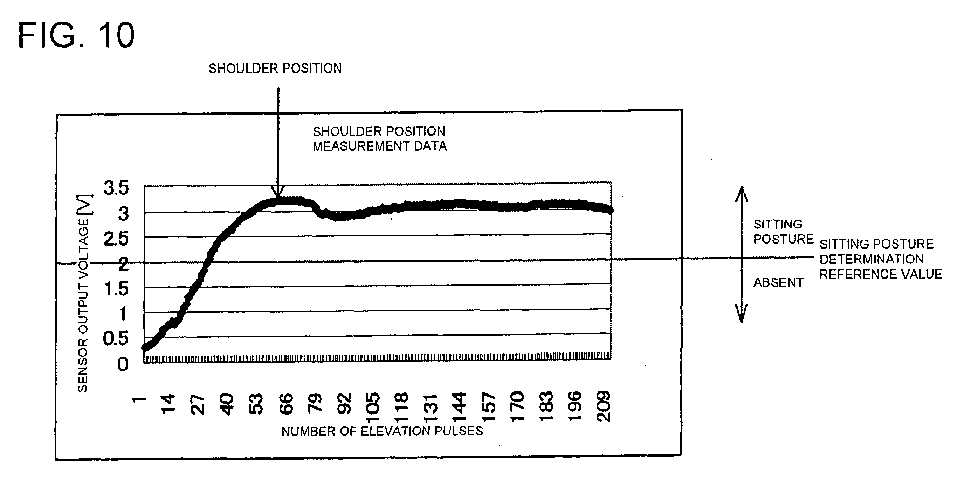

The treatment section 40, which has the massage head arms 403R and 403L, is driven by the elevating treatment unit 20 to descend from the upper portion to the lower portion of the back rest portion 10a in correspondence to the number of pulses of the motor. In the present embodiment, the signal is sensed on the basis of the number of elevation pulses of the motor, and a variance graph of the signal output voltage shown in FIG. 10 is obtained.

The sensed signal is serially supplied to the control section 13 of the massager 1, and the shoulder position, the presence or absence of the sitting posture, or the like is determined in the CPU 13a. In accordance with the information, the massage action is controlled.

The following provides description of measuring, such as sensing of the presence or absence of the sitting posture and the shoulder position and control of the massage action, which are carried out in the massager 1 according to the above-described embodiment. The description is made with reference to respective embodiment examples.

[First Embodiment Example]

(Sensing Method for Sitting Posture)

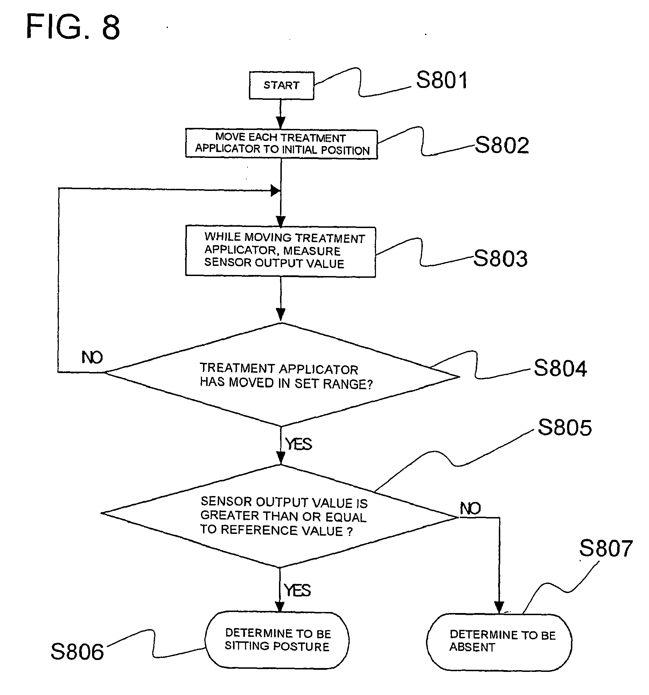

FIG. 8 is a flow diagram in the event of sensing the presence or absence of the sitting posture of a user in the massager.

In the massager 1 according to the embodiment, in the state where the massage action is not performed, the elevating treatment unit 20 is placed in a standby state in an upper portion of the back rest portion 10a so as to be prevented from abutment against the back of the user when the user takes the sitting posture on the massager 1.

When, for example, the user turns on the power supply of the massager 1 and depresses a start button of the massage action, the operation of the massager 1 starts (step S801).

Then, the elevating treatment unit 20, which has the massage heads (treatment applicators) 402a to 402d, starts movement to an initial position (step S802).

In association with the movement of the elevating treatment unit 20, the massage head arms 403R and 403L journaling the massage heads 402a to 402d starts slow movement from the upper portion to the lower portion. The sensor section 100 senses an impedance variance occurring between the massage head arms 403R and 403L, which function as the electrodes, as an output voltage variance (step S803).

Then, the control section 13 determines whether movement of the treatment section 40 in a predetermined measurement range has been completed (step S804). If the movement in the predetermined measurement range has not yet been completed ("NO"), then at step S803 the elevating treatment unit 20 is again moved, and the sensor section 100 senses an impedance variance. On the other hand, the movement in the predetermined measurement range has been completed ("YES"), the operation proceeds to step S805.

If output variances, as shown in FIG. 10 for example, are attained through iteration of steps S803 and S804, the CPU 13a makes a comparison between a predetermined reference value V11, which is prestored in storing means such as the ROM, and an output value V12 of a signal as a bio-signal from the sensor section 100. If the output value V12 is greater than or equal to the reference value V11 (which is set to 2.0 V in the present embodiment example), then it is determined that the user is in the sitting posture (step S806). On the other hand, if the output value V12 is not greater than or equal to the reference value V11 during the movement of the treatment applicators in the set range, then it is determined that the user is not in the sitting posture and hence absent (step S807).

As such, according to the massager of the present embodiment example, the presence or absence of the sitting posture of the user can be simply sensed.

In addition, since the massage head arms 403R and 403L are used as the electrodes, additional sensing-dedicated components are not necessary, therefore making it possible to provide low cost massagers having a simple configuration.

[Second Embodiment Example]

(Sensing Method for Shoulder Position)

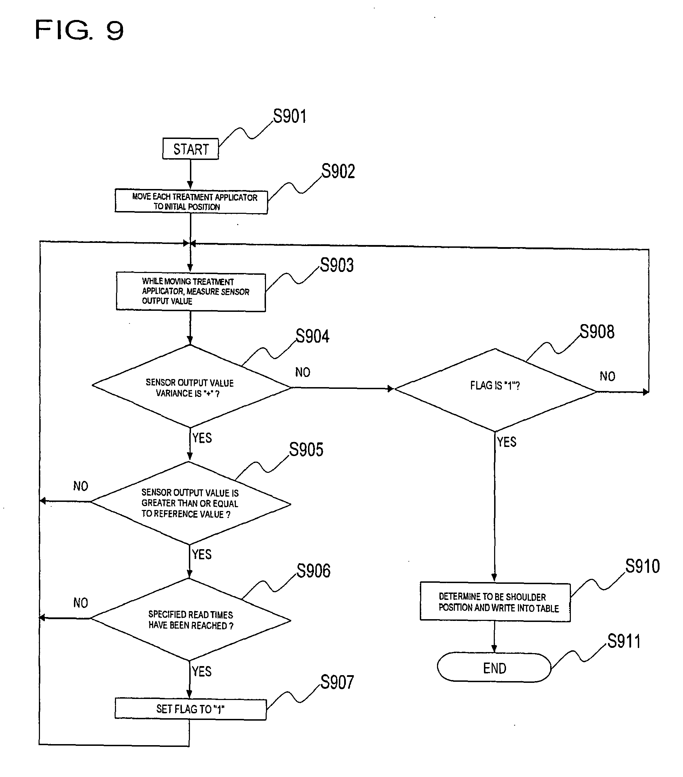

FIG. 9 is a flow diagram in the event of sensing the shoulder position of the user in the massager.

In the massager 1 according to the embodiment, in the state where the massage action is not performed, the treatment section 40 is placed in a standby state in an upper portion of the back rest portion 10a so as to be prevented from abutment against the back of the user when the user takes the sitting posture on the massager 1.

When, for example, the user turns on the power supply of the massager 1 and depresses a start button of the massage action, the operation of the massager 1 starts (step S901).

Then, the treatment section 40, which has the massage heads (treatment applicators) 402a to 402d, starts movement to an initial position (step S902).

In association with the movement of the treatment section 40, the massage head arms 403R and 403L journaling the massage heads 402a to 402d starts slow movement from the upper portion to the lower portion. The sensor section 100 senses an impedance variance occurring between the massage head arms 403R and 403L, which function as the electrodes, as an output voltage variance (step S903).

Then an output value V21 of the sensor section 100 is compared with a previous output value V20 of the sensor section 100 stored in the ROM 13b or RAM 13c (step S904). In the case of comparison of an initial output value, since no previous output value exists, the output value may be compared with an initial value ("0", for example) prestored in the storing means.

If the CPU 13a has determined that the output value V21 is increased from or has an increment (+) with respect to the output value V20 ( "YES" ), then the CPU 13a executes a comparison between the output value V21, which in the form of a signal corresponding to a bio-signal from the sensor section 100, and a predetermined reference value 22 prestored in, for example, the ROM 13b (step S905). This step is provided for the following reason. At an initial stage of measuring, a case can occur wherein the output value from the sensor section 100 does not simply increase, but vertically oscillates, so that the shoulder position determination is inhibited from being executed when the output value V21 is less or equal to the reference value 22. In other words, when an initial peak value of the output value from the sensor section 100 is not greater than or equal to the predetermined reference value, the shoulder position determination is not performed, thereby to prevent such a case where the shoulder position is incorrectly determined in an initial measurement zone wherein the output value from the sensor vertically oscillates, and hence the massager performs the massage action in accordance with the incorrect shoulder position.

If the output value V21 from the sensor section 100 is less than the reference value V22 ("NO"), then the operation returns to step S903, whereby the massage head arm 403R, 403L is moved by a predetermined distance, and the output value from the sensor section 100 is again measured.

On the other hand, if the output value V21 from the sensor section 100 is greater than or equal to the referencevalueV22 ( "YES" ), at step S906 it is determined whether the output value V21 has been read specified times N ("specified read times N, " hereafter). Thereby, even when the output value V21 of the sensor section 100 mistakenly takes a value satisfying steps S904 and S905 before reaching the shoulder position of the user, the determination of the shoulder position is prevented from being made before completion of measurement within a range preset from ordinary human body types.

The specified read times N in the present embodiment example is proportional to the number of pulses occurring in the event of driving the motor 17b that vertically moves the treatment section 40. Accordingly, if the number of measurements computed from the number of pulses occurring in the event of driving the motor 17b does not reach the specified read times N, the shoulder position sensing is not performed, and the operation returns to step S903.

On the other hand, if measurement times computed from the number of pulses in the event of driving the motor have reached the specified read times N, then at step S907 "1" is set to a flag to enable sensing of the shoulder position.

At step S904 if the CPU 13a has determined that the output value V21 is not increased from or has no increment with respect to the output value V20 ("NO"), then the CPU 13a determines whether the flag is set to "1" (step S908).

In the event the flag is not set to "1" ("NO"), at least one of steps S904 to S906 is not satisfied, so that the operation returns to step S903. Thereby, the massage head arms 403R and 403L is moved by a predetermined distance, and the output value from the sensor section 100 is again measured at that position.

On the other hand, in the event the flag is set to "1" ("YES"), the conditions of steps S904 to S906 are satisfied. As such, a position where the sensor output value stops increasing, that is, a position corresponding to the initial peak value of the signal corresponding to the bio-signal in the predetermined range upon the movement of the treatment section 40 is determined as the shoulder position, and written into a table (step S910).

As described above, the back face side of the user in the sitting posture on the back rest portion 10a is measured by moving the massage head arms 403R and 403L, which function as the electrodes, at predetermined pitch. Thereby, as shown in FIG. 10, the output voltage from the sensor section 100 can be obtained in the form of a curve having the peak in terms of the number of elevation pulses corresponding to the shoulder position. In addition, according to the present embodiment example, the shoulder position can be determined from the number of elevation pulses in the range of reaching the peak corresponding to the shoulder position.

Accordingly, according to the massager 1 of the present embodiment example, the shoulder position is automatically determined without requiring the user to set the shoulder position. Consequently, the accurate massage action can be performed without excessive burden being imposed on the user.

Further, the massage head arms 403R and 403L are used as the electrodes, additional sensing-dedicated components are not necessary, therefore making it possible to provide low cost massagers having a simple configuration.

Furthermore, sensing can be performed corresponding to the number of elevation pulses for driving the motor 17b that drives the massage head arms 403R and 403L to elevate (move up and down), high accuracy sensing of the shoulder position can be implemented.

[Third Embodiment Example]

(Sensing of Sitting posture in Massage Action Starting Process)

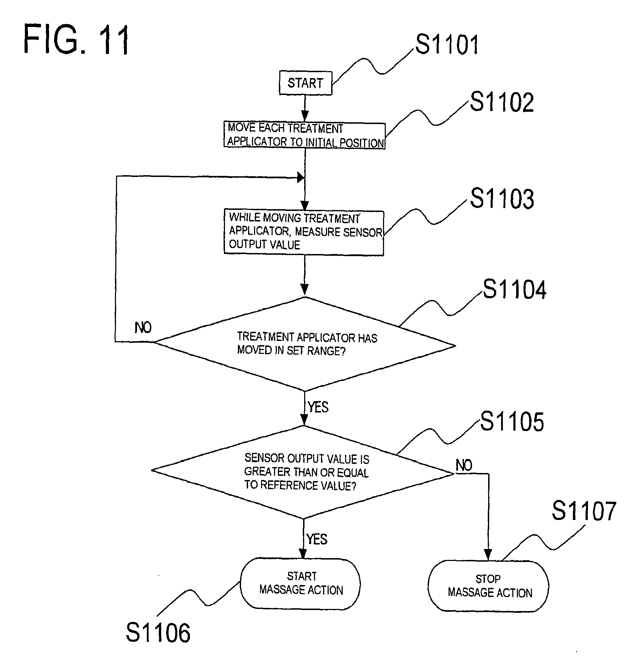

FIG. 11 is a flow diagram in the event of sensing the presence or absence of the sitting posture of the user in a massage action starting process in the massager.

Similarly as in the first embodiment example, in the massager 1 according to the embodiment, in the state where the massage action is not performed, the treatment section 40 is placed in a standby state in an upper portion of the back rest portion 10a so as to be prevented from abutment against the back of the user when the user takes the sitting posture on the massager 1.

When, for example, the user turns on the power supply of the massager 1 and depresses a start button of the massage action, the operation of the massager 1 starts (step S1101).

Then, the treatment section 40, which has the massage heads (treatment applicators) 402a to 402d, starts movement to an initial position (step S1102).

Subsequently, the presence or absence of the sitting posture of the user is sensed. Specifically, in association with the movement of the treatment section 40, the massage head arms 403R and 403L journaling the massage heads 402a to 402d starts slow movement from the upper portion to the lower portion. The sensor section 100 senses an impedance variance occurring between the massage head arms 403R and 403L, which function as the electrodes, as an output voltage variance (step S1103) .

Then, the control section 13 determines whether movement of the treatment section 40 in a predetermined measurement range has been completed (step S1104). If the movement in the predetermined measurement range has not yet been completed ("NO"), then at step S1103 the treatment section 40 is again moved, and the sensor section 100 senses an impedance variance. On the other hand, the movement in the predetermined measurement range has been completed ("YES"), the operation proceeds to step S1105.

In the event that output variances as shown in FIG. 10, for example, is attained through iteration of steps S1103 and S1104, the CPU 13a makes a comparison between a predetermined reference value V11, which is prestored in storing means such as the ROM, and an output value V12 of a signal as a bio-signal from the sensor section 100. If the output value V12 is greater than or equal to the reference value V11 (which is set to 2.0 V in the present embodiment example), then it is determined that the user is in the sitting posture and the massage action is started (step S1106).

On the other hand, if the output value V12 is not greater than or equal to the reference value V11 during the movement of the treatment applicators in the set range, then it is determined that the user is not in the sitting posture and hence absent, and the massage action is not started. Therefore, the operation of the massager 1 terminates (step S1107).

Accordingly, according to the configuration described above, even in the event that a user has mistakenly started the massage action, when the user is not in the sitting posture, the following massager action can be automatically stopped.

In addition, since the massage head arms 403R and 403L are used as the electrodes, additional sensing-dedicated components are not necessary, therefore making it possible to provide low cost massagers having a simple configuration.

[Fourth Embodiment Example]

(Sensing of Sitting posture in Automatic Course)

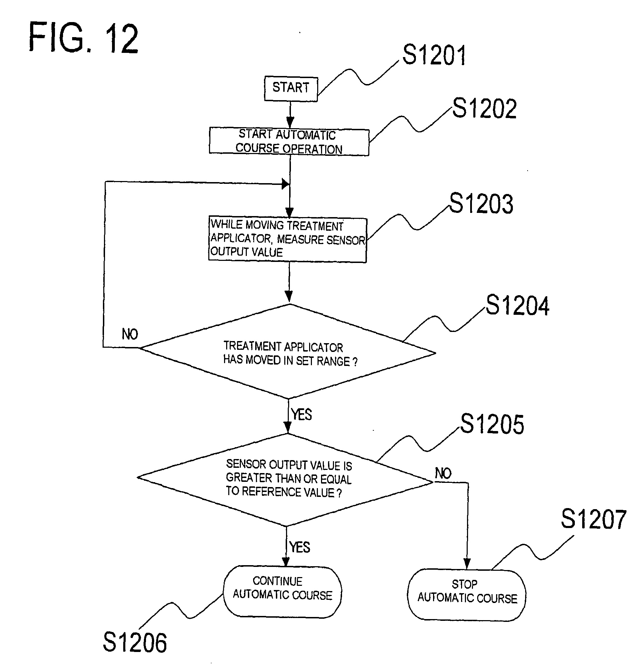

FIG. 12 is a flow diagram in the event of sensing the presence or absence of the sitting posture of the user in an automatic massage action of the massager.

When, for example, the user turns on the power supply of the massager 1 and depresses a start button for the automatic massage action, an automatic course operation of the massager 1 starts (step S1201, S1202).

Subsequently, in association with the movement of the treatment section 40, the massage head arms 403R and 403L journaling the massage heads 402a to 402d starts slowmovement from the upper portion to the lower portion. The sensor section 100 senses an impedance variance occurring between the massage head arms 403R and 403L, which function as the electrodes, as an output voltage variance (step S1203).

Then, the control section 13 determines whether movement of the treatment section 40 for a predetermined measurement range has been completed (step S1204). If the movement in the predetermined measurement range has not yet been completed ("NO"), then at step S1203 the treatment section 40 is again moved, and the sensor section 100 senses an impedance variance. On the other hand, the movement in the predetermined measurement range has been completed ("YES"), the operation proceeds to step S1205.

In the event that the output variances shown in FIG. 10, for example, are attained through iteration of steps S1203 and S1204, the CPU 13a makes a comparison between a predetermined reference value V11, which is prestored in storing means such as the ROM, and an output value V12 of a signal as a bio-signal from the sensor section 100. If the output value V12 is greater than or equal to the reference value V11 (which is set to 2.0 V in the present embodiment example), then it is determined that the user is in the sitting posture, and the automatic course is continued (step S1206). On the other hand, if the output value V12 is not greater than or equal to the reference value V11 during the movement of the treatment applicators in the set range, then it is determined that the user is not in the sitting posture and hence absent. Accordingly, the CPU 13a controls the massage action being performed with the treatment applicators to terminate, thereby to stop the automatic course (step S1207).

Thus, according to the massager 1 of the embodiment example, since the massage head arms 403R and 403L are used as the electrodes, sensing can be performed concurrently with the massage action being performed, and the presence or absence of the sitting posture of the user can be sensed in the course of the automatic course. Consequently, even when the user leaves the massager 1 in an intermediate stage, subsequent massage actions can be automatically stopped.

In addition, since the massage head arms 403R and 403L are used as the electrodes, additional sensing-dedicated components are not necessary, therefore making it possible to provide low cost massagers having a simple configuration.

[Fifth Embodiment Example]

(First Correction Method for Shoulder Position)

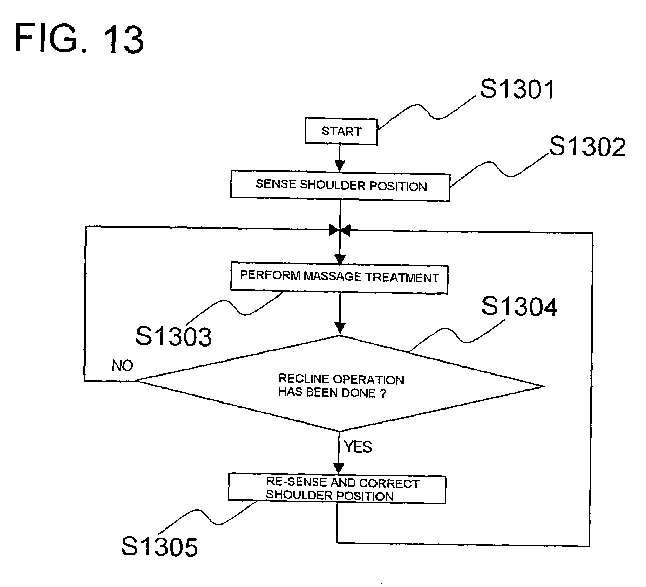

FIG. 13 is a flow diagram in the event of correction of the shoulder position of the user in the massager 1.

In the massager 1 according to the embodiment, upon start of the massage action (step S1301), the shoulder position is determined in accordance with, for example, the method described in conjunction with the second embodiment example (step S1302).

Subsequently, the massager 1 performs a massage treatment in accordance with the determined shoulder position of the user (step S1303). During the massage treatment, a case can occur where the user changes the tilted position of the back rest portion 10a (i.e., reclining) to take a more preferable posture. In this case, variations occur in the shoulder position of the user with respect to the back rest portion 10a. Consequently, appropriate massage action cannot be performed in the manner of performing the massage action in accordance with the previously determined shoulder position.

As such, in the present embodiment example, at step S1304 it is determined whether a recline operation has been performed. If the recline operation has not been performed ("NO"), since specific correction of the shoulder position is not necessary, the massage treatment is performed in accordance with the current setting of the shoulder position.

On the other hand, if it is determined that the recline operation has been performed ("YES"), then at step S1305 the shoulder position is again set. Specifically, sensing of the shoulder position may be again performed in the manner described in the second embodiment example, thereby to determine the shoulder position. In accordance with the shoulder position again determined, the control section 13 controls subsequent massage actions being performed with the treatment applicators.

Alternatively, from a movement angle (tilt) formed by the recline operation of the back rest portion 10a, the shoulder position variance may be computed and determined as a new shoulder position by using an operational expression prestored in the storing means or the like. Then, in accordance with the corrected shoulder position, the control section 13 controls subsequent massage actions being performed with the treatment applicators. According to the method described above, the treatment section 40 need not be moved back to the initial position to sense the shoulder position again, therefore enabling reduction in time necessary for subsequent massage treatments.

[Sixth Embodiment Example]

(Second Correction Method for Shoulder Position)

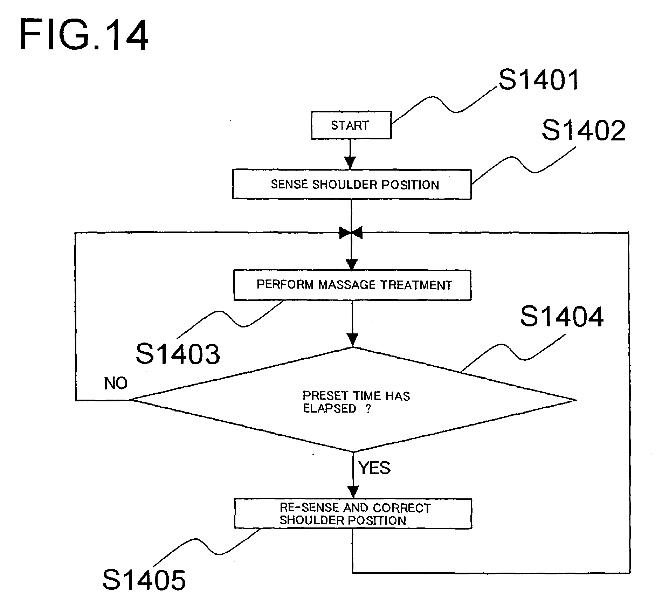

FIG. 14 is a flow diagram in the event of correction of the shoulder position of the user in the massager 1.

In the massager 1 according to the present embodiment, upon start of the massage action (step S1401), the shoulder position is determined in, for example, the manner described in conjunction with the second embodiment example (step S1402).

Subsequently, the massager 1 performs a massage treatment in accordance with the determined shoulder position of the user (step S1403). In passage of the time of the massage treatment, a case can occur where the user changes the posture and/or moves on the massager 1 to take a more preferable posture. In this case, variations occur in the shoulder position of the user with respect to the back rest portion 10a. Consequently, appropriate massage action cannot be performed in the manner of performing the massage action in accordance with the previously determined shoulder position.

As such, in the present embodiment example, re-sensing or correction of the shoulder position is performed in units of a preset time to accurately determine the shoulder position, thereby enabling an optimal massage action. Specifically, at step S1404 it is determined whether the preset time has elapsed. If the preset time has not elapsed, ("NO"), since specific correction of the shoulder position is not necessary, the massage treatment is performed in accordance with the current setting of the shoulder position.

On the other hand, if it is determined that the preset time has elapsed ("YES"), then at step S1405 the shoulder position is again set and corrected. Specifically, sensing of the shoulder position may be again performed in the manner described in the second embodiment example, thereby to determine the shoulder position. In accordance with the shoulder position again determined, the control section 13 controls massage actions being performed with the treatment applicators. According to the method described above, the shoulder position can be accurately determined at all times, optimal massage actions can be performed.

Thus, although the present invention has been described with reference to the embodiment and respective embodiment examples, the present invention is not limited to the respective embodiment and embodiment examples. However, the present invention may, of course, be modified and altered in various ways and be configured by optionally combining the embodiment and embodiment examples as long as permissible in accordance with scope of the invention.

**Claims**

1. A massager comprising:

   a back rest portion that supports a back side of a user;
   treatment applicators that are provided to the back rest portion and that massage the user;
   a treatment section that moves a pair of electrodes supporting the treatment applicators, in association with a massage action;
   a bio-signal sensor that senses a bio-signal of the user from an impedance variance between the pair of electrodes; and
   a control section that controls the massage action of the treatment applicators in accordance with the bio-signal, wherein
   the bio-signal sensor includes:

      an oscillation section that supplies a high frequency signal;
      the pair of electrodes that are disposed to allow the user to near or contact and that are provided to receive the high frequency signal from the oscillator section;
      a sensor section that senses the impedance variance occurring between the electrodes having received the high frequency signal when the user has neared and contacted the electrodes; and
      a signal processor section that processes a signal corresponding to the sensed impedance variance into a desired signal corresponding to the bio-signal,
      wherein the control section makes a comparison between the signal corresponding to the bio-signal and a predetermined reference value, thereby to determine presence or absence of a sitting posture of the user.

2. A massager according to claim 1, wherein
   when the signal corresponding to the bio-signal is not greater than or equal to the predetermined reference value during movement of the treatment applicators in a set range, the control section controls the massage action with the treatment applicators to not start.

3. A massager according to claim 1, wherein
   when the signal corresponding to the bio-signal is not greater than or equal to the predetermined reference value during movement of the treatment applicators in a set range, the control section controls the massage action being performed with the treatment applicators to stop.

4. A massager according to any one of claims 1 to 3, wherein
   the electrodes are pivotable with respect to the treatment section, and are arms each supporting a plurality of the treatment applicators; and
   the plurality of treatment applicators are provided to be aligned substantially parallel to a movement direction of the treatment section in a manner to sandwich a pivoting center of the arm.

**5.** A massager comprising:

a back rest portion that supports a back side of a user;

treatment applicators that are provided to the back rest portion and that massage the user;

a treatment section that moves a pair of electrodes supporting the treatment applicators, in association with a massage action;

an elevation section that elevationally move the treatment section within the back rest portion;

a bio-signal sensor that senses a bio-signal of the user from an impedance variance between the pair of electrodes; and

a control section that controls the massage action of the treatment applicators in accordance with the bio-signal, wherein

the bio-signal sensor includes:

an oscillator section that supplies a high frequency signal;

the pair of electrodes that are disposed to allow the user to near or contact and that are provided to receive the high frequency signal from the oscillator section;

a sensor section that senses the impedance variance occurring between the electrodes having received the high frequency signal when the user has neared and contacted the electrodes; and

a signal processor section that processes a signal corresponding to the sensed impedance variance into a desired signal corresponding to the bio-signal,

wherein the control section causes the treatment section to move and detects an extreme value of the signal corresponding to the bio-signal in a predetermined range, thereby to determine a shoulder position of the user.

**6.** A massager according to claim 5, wherein
the extreme value is an initial peak value.

**7.** A massager according to claim 6, wherein
when the initial peak value is not greater than or equal to a predetermined reference value, the control section does not determine the shoulder position of the user.

**8.** A massager according to any one of claims 5 to 7, wherein
when a tilt of the back rest portion is altered, the control section again determines the shoulder position and controls the massage action with the treatment applicators in accordance with the shoulder position again determined.

**9.** A massager according to any one of claims 5 to 7, wherein
when a tilt of the back rest portion is altered, the control section controls the massage action with the treatment applicators in accordance with the shoulder position corrected corresponding to the tilt.

**10.** A massager according to any one of claims 5 to 7, wherein
after a predetermined time has elapsed since the control section determined the shoulder position, the control section again determines the shoulder position and controls the massage action with the treatment applicators in accordance with the shoulder position again determined.

**11.** A massager according to any one of claims 5 to 10, wherein
the electrodes are pivotable with respect to the treatment section, and are arms each supporting a plurality of the treatment applicators; and
the plurality of treatment applicators are provided to be aligned substantially parallel to a movement direction of the treatment section in a manner to sandwich a pivoting center of the arm.

**12.** A massager according to any one of claims 1 to 11, wherein
the sensor section generates and supply a signal that represents a variance in a level of a reflected wave of the high frequency signal and that corresponds to the impedance variance occurring between the electrodes in correspondence to a body type of the user.

**13.** A massager according to any one of claims 1 to 11, wherein
the sensor section outputs reflection wave signals corresponding to real and imaginary impedance variances occurring between the electrodes in correspondence to a body type of the user.

**14.** A massager according to any one of claims 1 to 13, wherein
the frequency of the high frequency signal is in a range of from 10 MHz to 300 MHz.

# FIG. 1

EP 1 629 816 A1

FIG. 2

402a

402b

402c

402d

1

20

10a

10b

10

FIG. 3

FIG. 4

# FIG. 5

## FIG. 6

# FIG. 7

# FIG. 8

START — S801

MOVE EACH TREATMENT
APPLICATOR TO INITIAL POSITION — S802

WHILE MOVING TREATMENT
APPLICATOR, MEASURE
SENSOR OUTPUT VALUE — S803

TREATMENT APPLICATOR
HAS MOVED IN SET RANGE? — S804

NO

YES

SENSOR OUTPUT VALUE IS
GREATER THAN OR EQUAL
TO REFERENCE VALUE ? — S805

NO

YES

S806 — DETERMINE TO BE
SITTING POSTURE

DETERMINE TO BE
ABSENT — S807

# FIG. 9

START — S901

MOVE EACH TREATMENT APPLICATOR TO INITIAL POSITION — S902

WHILE MOVING TREATMENT APPLICATOR, MEASURE SENSOR OUTPUT VALUE — S903

SENSOR OUTPUT VALUE VARIANCE IS "+" ? — S904
NO → FLAG IS "1"? — S908
NO →

YES ↓

SENSOR OUTPUT VALUE IS GREATER THAN OR EQUAL TO REFERENCE VALUE ? — S905
NO ←

YES ↓

SPECIFIED READ TIMES HAVE BEEN REACHED ? — S906
NO ←

YES ↓

SET FLAG TO "1" — S907

FLAG IS "1"? YES ↓

DETERMINE TO BE SHOULDER POSITION AND WRITE INTO TABLE — S910

END — S911

# FIG. 10

SHOULDER POSITION

SHOULDER POSITION
MEASUREMENT DATA

SITTING
POSTURE

ABSENT

SITTING POSTURE
DETERMINATION
REFERENCE VALUE

EP 1 629 816 A1

# FIG. 11

S1101

START

S1102

MOVE EACH TREATMENT
APPLICATOR TO INITIAL POSITION

S1103

WHILE MOVING TREATMENT
APPLICATOR, MEASURE SENSOR
OUTPUT VALUE

S1104

NO ← TREATMENT APPLICATOR HAS
MOVED IN SET RANGE?

YES

S1105

SENSOR OUTPUT VALUE IS
GREATER THAN OR EQUAL
TO REFERENCE VALUE? → NO

S1107

YES

START
MASSAGE ACTION

STOP
MASSAGE ACTION

S1106

# FIG. 12

```
                    START  ────── S1201
                      │
                      ▼
            START AUTOMATIC   ────── S1202
            COURSE OPERATION
                      │
            ┌─────────┤
            │         ▼
            │  WHILE MOVING TREATMENT   ────── S1203
            │  APPLICATOR, MEASURE SENSOR
            │  OUTPUT VALUE
            │         │
            │         ▼
            │    ◇ TREATMENT APPLICATOR ◇  ────── S1204
       NO   │    ◇ HAS MOVED IN SET RANGE ? ◇
       └────┘         │
                    YES
                      ▼
            ◇ SENSOR OUTPUT VALUE IS ◇   ────── S1205
            ◇ GREATER THAN OR EQUAL ◇  NO
            ◇ TO REFERENCE VALUE ? ◇ ─────┐
                      │                    │
                    YES                    ▼
                      ▼            ( STOP         )  ────── S1207
            ( CONTINUE      )     ( AUTOMATIC COURSE )
            ( AUTOMATIC COURSE )

                S1206
```

# FIG. 13

S1301

START

S1302

SENSE SHOULDER POSITION

PERFORM MASSAGE TREATMENT

S1303

NO

S1304

RECLINE OPERATION
HAS BEEN DONE ?

YES

RE-SENSE AND CORRECT
SHOULDER POSITION

S1305

# FIG.14

START — S1401

SENSE SHOULDER POSITION — S1402

PERFORM MASSAGE TREATMENT

S1403

PRESET TIME HAS ELAPSED ? — S1404

NO

YES

RE-SENSE AND CORRECT SHOULDER POSITION

S1405

**European Patent Office**

**EUROPEAN SEARCH REPORT**

Application Number

EP 05 01 8706

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 01/37729 A (OMRON CORPORATION; KASAI, EIJI; FUKUI, RYO) 31 May 2001 (2001-05-31) * figures 1-6 * | 1-14 | A61H1/00 A61H37/00 A61B5/053 |
| A | PATENT ABSTRACTS OF JAPAN vol. 2000, no. 15, 6 April 2001 (2001-04-06) -& JP 2000 342644 A (YA MAN LTD), 12 December 2000 (2000-12-12) * abstract; figures * | 1-14 | |
| A | EP 1 230 904 A (SANYO ELECTRIC CO., LTD) 14 August 2002 (2002-08-14) * the whole document * | 1-14 | |
| A | US 2002/123704 A1 (HORI KUNIHIKO ET AL) 5 September 2002 (2002-09-05) * the whole document * | 1-14 | |
| A | EP 0 989 671 A (OMRON HEALTHCARE CO., LTD) 29 March 2000 (2000-03-29) * the whole document * | 1-14 | TECHNICAL FIELDS SEARCHED (IPC) A61H A61B |

The present search report has been drawn up for all claims

| Place of search | Date of completion of the search | Examiner |
|---|---|---|
| Munich | 23 November 2005 | Elmar Fischer |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**                    EP 05 01 8706

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2005

| Patent document cited in search report | | Publication date | Patent family member(s) | | Publication date |
|---|---|---|---|---|---|
| WO 0137729 | A | 31-05-2001 | AU<br>CN<br>JP | 1416301 A<br>1391450 A<br>3575463 B2 | 04-06-2001<br>15-01-2003<br>13-10-2004 |
| JP 2000342644 | A | 12-12-2000 | NONE | | |
| EP 1230904 | A | 14-08-2002 | CN<br>JP<br>TW<br>US | 1368040 A<br>2002233558 A<br>508239 B<br>2002138023 A1 | 11-09-2002<br>20-08-2002<br>01-11-2002<br>26-09-2002 |
| US 2002123704 | A1 | 05-09-2002 | TW | 510789 B | 21-11-2002 |
| EP 0989671 | A | 29-03-2000 | JP<br>JP<br>US | 3484355 B2<br>2000098048 A<br>6545614 B1 | 06-01-2004<br>07-04-2000<br>08-04-2003 |

EPO FORM P0459

For more details about this annex : see Official Journal of the European Patent Office, No. 12/82